(19) Europäisches Patentamt
European Patent Office
Office européen des brevets

(11) Veröffentlichungsnummer: **0 035 707**
**B1**

(12) # EUROPÄISCHE PATENTSCHRIFT

(45) Veröffentlichungstag der Patentschrift: **18.05.83**

(51) Int. Cl.³: **C 07 C 103/88,** C 07 C 102/08

(21) Anmeldenummer: **81101404.2**

(22) Anmeldetag: **26.02.81**

(54) **Verfahren zur Herstellung von alpha-Ketocarbonsäure-N-acylamiden.**

(30) Priorität: **08.03.80 DE 3009044**

(43) Veröffentlichungstag der Anmeldung: **16.09.81 Patentblatt 81/37**

(45) Bekanntmachung des Hinweises auf die Patenterteilung: **18.05.83 Patentblatt 83/20**

(84) Benannte Vertragsstaaten: **AT BE CH DE FR GB IT LI NL**

(56) Entgegenhaltungen: **DE-A-2 705 048**

(73) Patentinhaber: **BAYER AG, Zentralbereich Patente, Marken und Lizenzen, D-5090 Leverkusen 1, Bayerwerk (DE)**

(72) Erfinder: **Bonse, Gerhard, Dr., Wolfskaul 3, D-5000 Köln 80 (DE)**
*Erfinder:* **Blank, Heinz-Ulrich, Dr., Am Geusfelde 35, D-5068 Odenthal (DE)**

EP 0 035 707 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von α-Ketocarbonsäure-N-acylamiden

Die vorliegende Erfindung betrifft ein neuartiges Verfahren zur Herstellung von neuen α-Ketocarbonsäure-N-acylamiden, welche als Zwischenprodukte zur Synthese von bekannten herbiziden Wirkstoffen verwendet werden können.

Allgemein sind α-Ketocarbonsäureamide wertvolle Zwischenprodukte zur Herstellung von herbizid wirksamen 1,2,4-Triazin-5-on-Derivaten, die z.B. gemäss DE-OS 2 165 554 aus α-Ketocarbonsäureamiden und Hydrazinderivaten gut zugänglich sind.

Es ist bereits bekanntgeworden, Nitrile mit Carbonsäuren bzw. deren Anhydriden in Gegenwart von Katalysatoren, wie z.B. Mineralsäuren, zu N-acylsubstituierten Carbonsäureamiden umzusetzen (vgl. z.B. Compr. Org. Chem. 2, S. 539 (1979); «The Chemistry of the Cyano Group», Z. Rappoport, Interscience Publ., New York (1970), S. 239-305; Russ. Chem. Rev. 29, S. 331 (1960); Russ. Chem. Rev. 31, S. 615 (1962)).

So führt z.B. die Umsetzung von Propionitril mit Propionsäure bzw. Propionsäureanhydrid in Gegenwart von Schwefelsäure zu Dipropionamid, in einer Ausbeute von 28% d.Th. (vgl. J., Amer. Chem. Soc. 80, S. 376 (1958)).

Es ist weiterhin bekannt, dass Umsetzungen von Nitrilen mit Carbonsäuren bzw. Carbonsäureanhydriden in Gegenwart von Katalysatoren verschiedenartige Folgereaktionen eingehen können. So ergibt z.B. die Umsetzung von Acetonitril mit Essigsäureanhydrid in Gegenwart von HCl-Gas das Hydrochlorid des Acetamids und Acetylchlorid (Compt. rend, 121, S. 1155 (1895)), während beim Erhitzen dieser Komponenten auf 200°C Triacetamid gebildet wird.

Häufig erfolgt unter entsprechenden Reaktionsbedingungen im Sinne von Gleichgewichtsreaktionen auch ein Austausch der Nitrilgruppe und Carboxylgruppe tragenden Reste (vgl. Russ. Chem. Rev. 29, S. 331 (1960)).

Während die Darstellung von N-Acylamiden für eine Reihe von aliphatischen und aromatischen Nitrilen beschrieben ist, ist eine entsprechende Umwandlung in der Klasse der Acylcyanide zu α-Ketocarbonsäure-N-acylamiden bisher nicht bekannt.

Bisher war lediglich bekannt, dass in dieser Stoffklasse Benzoylcyanid unter speziellen acylierenden Bedingungen, mit Essigsäureanhydrid in Gegenwart von Natriumacetat, über eine C-Acylierung überwiegend zu Acetophenon und anderen Produkten umgesetzt wird (vgl. Liebigs Ann. Chem. 491, S. 264 (1931)).

Es wurde nun überraschend gefunden, dass man die neuen α-Ketocarbonsäure-N-acylamide der allgemeinen Formel I

$$R^1\text{-CO-CO-NH-CO-}R^2 \qquad (I)$$

in welcher
$R^1$ für einen gegebenenfalls substituierten aliphatischen Rest mit bis zu 12 Kohlenstoffatomen, für einen gegebenenfalls substituierten Cycloalkylrest mit 3 bis 10 Kohlenstoffatomen, für einen gegebenenfalls substituierten Phenyl- oder Naphthylrest oder für einen gegebenenfalls substituierten heterocyclischen Rest steht und
$R^2$ für einen gegebenenfalls substituierten aliphatischen Rest mit bis zu 8 Kohlenstoffatomen oder für einen gegebenenfalls substituierten Phenylrest steht,
erhält, wenn man Acylcyanide der allgemeinen Formel II

$$R^1\text{-CO-CN} \qquad (II)$$

in welcher
$R^1$ die oben angegebene Bedeutung hat,
mit Carbonsäureanhydriden der allgemeinen Formel III

$$R^2\text{-CO-O-CO-}R^2 \qquad (III)$$

in welcher
$R^2$ die oben angegebene Bedeutung hat,
in Gegenwart einer starken Säure und gegebenenfalls in Gegenwart eines Lösungsmittels bei Temperaturen zwischen −50 und +150°C umsetzt und das Reaktionsgemisch anschliessend mit Wasser versetzt.

Verwendet man Pivaloylcyanid und Essigsäureanhydrid als Ausgangsstoffe und führt die Umsetzung in Gegenwart von konzentrierter Schwefelsäure durch, so kann der Reaktionsablauf durch das folgende Formelschema wiedergegeben werden:

$$(CH_3)_3C\text{-CO-CN} \xrightarrow[\text{2) } H_2O]{\text{1) } (CH_3CO)_2O/H_2SO_4}$$

$$(CH_3)_3C\text{-CO-CO-NH-CO-}CH_3$$

Die als Ausgangsstoffe einzusetzenden Acylcyanide sind durch Formel (II) allgemein definiert. In dieser Formel steht $R^1$ vorzugsweise für geradkettiges oder verzweigtes Alkyl mit 1 bis 4 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Nitril und/oder Halogen, wie zum Beispiel Fluor, Chlor, Brom oder Jod; ferner steht $R^1$ vorzugsweise für gegebenenfalls durch Alkyl, Alkoxy oder Carbalkoxy mit jeweils bis zu 4 Kohlenstoffatomen, Nitro, Nitril und/oder Halogen, wie zum Beispiel Fluor, Chlor und Brom, substituiertes Cycloalkyl mit 3 bis 6 Kohlenstoffatomen im Ringsystem. Weiterhin steht $R^1$ vorzugsweise für gegebenenfalls durch Alkyl, Alkoxy oder Carbalkoxy mit jeweils bis zu 4 Kohlenstoffatomen, Nitro und/oder Halogen, wie zum Beispiel Fluor, Chlor und Brom,

substituiertes Phenyl oder Naphthyl; schliesslich steht $R^1$ vorzugsweise für gegebenenfalls durch Alkyl, Alkoxy oder Carbalkoxy mit jeweils bis zu 4 Kohlenstoffatomen, Nitro, Nitril und/oder Halogen, wie z.B. Fluor, Chlor und Brom, substituierte 5- oder 6-gliedrige heterocyclische Reste, die 1 bis 3 Heteroatome wie Sauerstoff, Schwefel und/oder Stickstoff im Ring enthalten können und ausserdem mit einem Benzolring annelliert sein können. Als Beispiele für insbesondere in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Die Acylcyanide der Formel (II) sind zum Teil bekannt; noch nicht bekannte Acylcyanide können nach bekannten Verfahren hergestellt werden (vgl. Angew. Chem, 68, S. 425-435 (1965); ferner DE-OS'en 2 614 240, 2 614 241, 2 614 242, 2 708 182, 2 708 183).

Als im Rahmen dieser Erfindung besonders bevorzugte Acylcyanide seien Pivaloylcyanid und Benzoylcyanid genannt.

Die weiterhin als Ausgangsstoffe einzusetzenden Carbonsäureanhydride sind durch Formel (III) allgemein definiert. In dieser Formel steht $R^2$ vorzugsweise für gegebenenfalls chlorsubstituiertes Alkyl mit 1 bis 4 Kohlenstoffatomen oder für Phenyl.

Die Carbonsäureanhydride der Formel (III) sind zum Teil grosstechnisch verfügbar bzw. nach allgemein bekannten Methoden z.B. aus den entsprechenden Carbonsäuren herstellbar.

Im Rahmen dieser Erfindung besonders bevorzugte Carbonsäureanhydride sind Essigsäureanhydrid, Propionsäureanhydrid und die Anhydride der Chloressigsäuren.

Die erfindungsgemässe Umsetzung wird in Gegenwart einer starken Säure durchgeführt. Als solche Säuren kommen anorganische Säuren, wie konzentrierte Schwefelsäure, Salpetersäure, Perchlorsäure und Phosphorsäure, ferner Lewis-Säuren wie Bortrifluorid, Aluminiumchlorid oder Zinkchlorid in Frage. Weiterhin geeignet sind aliphatische und aromatische Sulfon- und Phosphonsäuren sowie Halogenalkancarbonsäuren wie z.B. Trichloressigsäure. Bevorzugt werden Sauerstoffsäuren, insbesondere wird konzentrierte Schwefelsäure verwendet.

Es ist möglich, die erfindungsgemässe Umsetzung in Gegenwart einer oder mehrerer solcher Säuren durchzuführen.

Die Reaktionstemperaturen können in einem grösseren Bereich variiert werden. Im allgemeinen arbeitet man, wie oben angegeben, bei Temperaturen zwischen etwa −50 und +150°C, vorzugsweise zwischen etwa 0 und 100°C. Die anschliessende Aufarbeitung wird zweckmässig mittels Eiswasser durchgeführt.

Die Reaktion wird im allgemeinen bei Normaldruck durchgeführt.

Die Umsetzung kann in Abwesenheit oder in Gegenwart eines Lösungsmittels bzw. Lösungsvermittlers durchgeführt werden. Als Lösungsvermittler kommen bestimmte organische Lösungsmittel in Frage; besonders geeignet sind Eisessig und Dichlormethan, ferner Dialkylether, wie Diethyl- oder Di-isopropylether, und Diarylether, wie z.B. Diphenylether.

Bei der Durchführung des erfindungsgemässen Verfahrens setzt man im allgemeinen auf 1 Mol Acylcyanid der Formel (II) 0,5 bis 10 Mol, vorzugsweise 0,8 bis 4 Mol Carbonsäureanhydrid der Formel (III) ein; besonders bevorzugt ist ein Molverhältnis Acylcyanid (II) zu Carbonsäureanhydrid (III) von 1:1 bis 1:2.

Die für die Durchführung des erfindungsgemässen Verfahrens erforderlichen Säuren werden in katalytischen bis überstöchiometrischen Mengen eingesetzt. Im allgemeinen setzt man auf 1 Mol Acylcyanid (II) 0,5 bis 10 Mol, vorzugsweise 0,8 bis 8 Mol, besonders bevorzugt 1 bis 4 Mol Säure ein.

Besonders vorteilhaft ist ein Molverhältnis Carbonsäureanhydrid (III) zu Säure von 1:2.

Damit ergibt sich, dass ein Molverhältnis Acylcyanid (II) zu Carbonsäureanhydrid (III) zur Säure von 1:1:2 bis 1:2:4 ganz besonders günstig ist.

Zweckmässigerweise geht man bei der Durchführung des Verfahrens so vor, dass man die Säure und Carbonsäureanhydrid (III) bzw. ein Gemisch aus Lösungsmittel, Säure und Carbonsäureanhydrid (III) vorlegt und Acylcyanid (II), gegebenenfalls in Lösungsmittel, zugibt.

Die Reaktionszeiten betragen im allgemeinen 1–10 Stunden. Das Reaktionsgemisch wird anschliessend am zweckmässigsten auf Eis gegossen. Die gebildeten α-Ketocarbonsäure-N-acylamide können durch Filtration oder durch Extraktion isoliert werden.

Hierzu geeignete Extraktionsmittel sind mit Wasser nicht beliebig mischbare Lösungsmittel, beispielsweise Ether, wie Diethylether oder Diisopropylether, Ester wie z.B. Essigsäureethylester, Ketone wie z.B. Methylisobutylketon, Halogenkohlenwasserstoffe wie z.B. Dichlormethan, Chlorbenzol oder Dichlorbenzol, ferner Aromaten wie z.B. Benzol, Toluol, o-Xylol, Ethylbenzol, Cumol oder Nitrobenzol. Vorzugsweise verwendet man Dichlormethan.

Die erfindungsgemäss herstellbaren α-Ketocarbonsäure-N-acylamide der Formel (I) sind neu und können z.B. als Zwischenprodukte zur Synthese von herbiziden Wirkstoffen verwendet werden. So erhält man beispielsweise aus dem Trimethylbrenztraubensäure-N-acetylamid (Ia) nach folgendem Reaktionsschema die herbizid besonders wirksame Verbindung 6-Amino-6-tert.-butyl-3-metyl-thio-1,2,4-triazin-5(4H)-on (VI) (vgl. DE-PS 1 795 784):

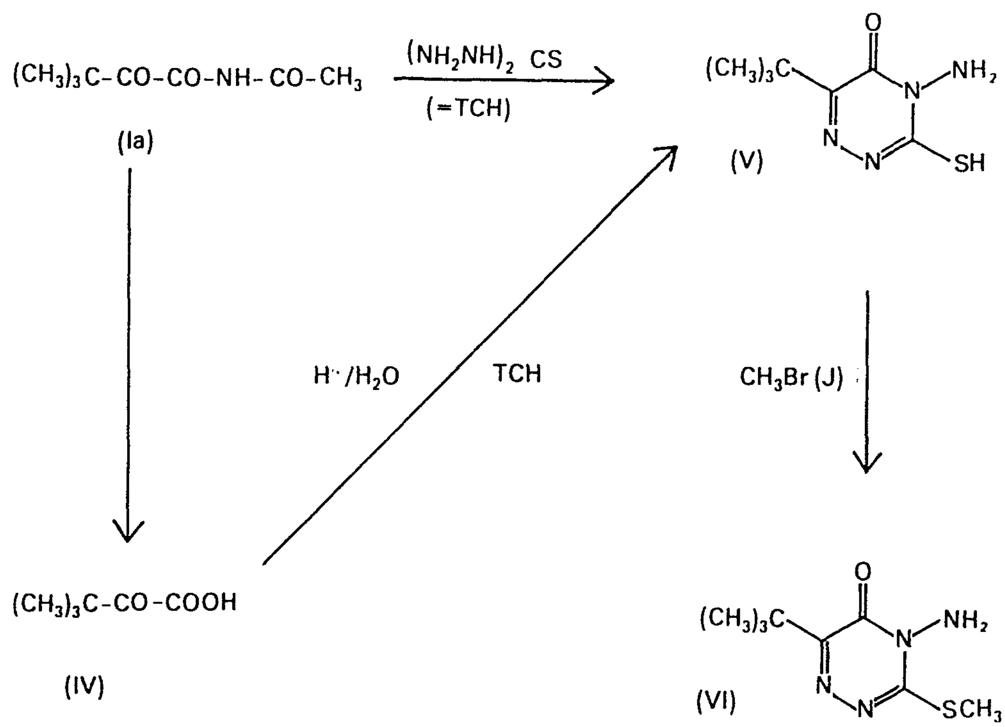


(Ia) (IV) (V) (VI)

Trimethylbrenztraubensäure-N-acetylamid (Ia) kann entweder direkt oder nach vorheriger Verseifung zur freien α-Ketosäure (IV) in saurer, wässriger Lösung oder Suspension mit 1 bis 1,5 Mol Thiocarbohydrazid, $NH_2$-NH-CS-NH-$NH_2$ (=TCH), bei Temperaturen zwischen −20 und +150°C zu 4-Amino-6-tert.-butyl-3-mercapto-1,2,4-triazin-5(4H)-on (V) kondensiert werden, welches sich beispielsweise mittels Methylhalogenid, z.B. Methyljodid oder Methylbromid, in alkalischer Lösung zu (VI) methylieren lässt (vgl. Chem. Ber. 97, S. 2173-8 (1964); DE-OS 2 165 554; DE-OS 2 460 889; DE-OS 2 648 300; DE-OS 2 733 180).

Das hier angegebene, über die erfindungsgemässen, neuen α-Ketocarbonsäure-N-acylamide verlaufende Verfahren zur Herstellung von herbizid wirksamen asymmetrischen Triazinonen vom Typ (VI) ist dem vergleichbaren vorbekannten, über α-Ketocarbonsäure-N-tert.-butylamide verlaufenden Verfahren (vgl. DE-OS 2 733 180 und DE-OS 2 733 181) technisch überlegen. Insbesondere können die erfindungsgemässen α-Ketocarbonsäure-N-acylamide (I) mit Thiocarbohydrazid unter sehr milden Bedingungen nahezu quantitativ zu asymmetrischen Triazinonen, die unmittelbar in hoher Reinheit anfallen, cyclisiert werden, während die vorbekannten α-Ketocarbonsäure-N-tert.-butylamide hierzu mit Thiocarbohydrazid mehrere Stunden auf 100°C erhitzt werden müssen und lediglich Ausbeuten von ca. 70% ergeben.

α-Ketocarbonsäure-N-acylamide stellen somit eine neue, wertvolle Klasse von Zwischenprodukten z.B. zur Synthese von α-Ketocarbonsäuren und von 1,2,4-Triazin-5-on-Derivaten dar.

Die nachfolgenden Herstellungsbeispiele sollen zur näheren Erläuterung der Erfindung dienen.

Herstellungsbeispiele

A) Herstellung von α-Ketocarbonsäure-N-acylamiden (I)

Beispiel 1

$(CH_3)_3C$-CO-CO-NH-CO-$CH_3$

In 49,0 g (0,5 Mol) vorgelegte konzentrierte Schwefelsäure werden jeweils bei Raumtemperatur zunächst 25,6 g (0,25 Mol) Essigsäureanhydrid und anschliessend 27,8 g (0,25 Mol) Pivaloylcyanid eingetragen. Nach 4-stündigem Nachrühren wird die Reaktionsmischung mit 150 g Eiswasser versetzt und gut durchgerührt. Das ausfallende Reaktionsprodukt wird abgesaugt, mit 100 ml Wasser gewaschen und getrocknet. Man erhält 37,0 g (86,5% d.Th.) Trimethylbrenztraubensäure-N-acetylamid als farblose glänzende Blättchen vom Schmelzpunkt 82-84°C; Gehalt nach gaschromatographischer Bestimmung >99%. Für weitere Umsetzungen sind keine zusätzlichen Reinigungsoperationen erforderlich.

Analyse:

$C_8H_{13}NO_3$ (MG = 171,2)

| | | | |
|---|---|---|---|
| Ber.: | C 56,13 | H 7,65 | N 8,18 |
| Gef.: | C 56,10 | H 7,85 | N 8,30 |

Beispiel 2

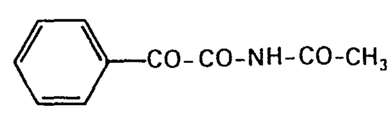

Arbeitet man analog zu Beispiel 1 und setzt an-

stelle von Pivaloylcyanid Benzoylcyanid ein, so erhält man 38,5 g (79,5% d.Th.) Phenylglyoxylsäure-N-acetylamid als farblose Kristalle vom Schmelzpunkt 124–125°C; Gehalt nach gaschromatographischer Bestimmung > 99%. Für weitere Umsetzungen sind keine zusätzlichen Reinigungsoperationen erforderlich.

Analyse:

$C_{10}H_9NO_3$ (MG = 191,2)

| | | | |
|---|---|---|---|
| Ber.: | C 62,82 | H 4,74 | N 7,33 |
| Gef.: | C 62,90 | H 4,70 | N 7,50 |

B) Folgereaktionen

1. Cyclisierung mit Thiocarbohydrazid/Methylierung

a) 4-Amino-6-tert.-butyl-3-mercapto-1,2,4-triazin-5(4H)-on (V):

Zu 3,2 g (0,03 Mol) Thiocarbohydrazid in 50 ml 1 n HCl lässt man 5,1 g (0,03 Mol) Trimethylbrenztraubensäure-N-acetylamid in 20 ml Ethanol zutropfen und lässt die Reaktionsmischung 5 Stunden lang bei Raumtemperatur nachrühren. Das ausgefallene Produkt wird abgesaugt, mit Wasser gewaschen und getrocknet. Man erhält 5,7 g des oben bezeichneten Produktes (V) vom Schmelzpunkt 210°C, mit einem gaschromatographisch bestimmten Gehalt von > 99%, was einer Ausbeute von 95% d.Th. entspricht.

b) 4-Amino-6-tert.-butyl-3-methylthio-1,2,4-triazin-5(4H)-on (VI):

In eine Mischung aus 97 g 45%iger Natronlauge und 65 g Wasser werden unter Rühren 20 g (0,1 Mol) 4-Amino-6-tert.-butyl-3-mercapto-1,2,4-triazin-5(4H)-on (V) eingetragen. Nach vollständiger Lösung des Produktes werden 16,5 g Methyljodid so zugegeben, dass die Innentemperatur 30°C nicht übersteigt. Nach beendeter Zugabe wird das Reaktionsgemisch noch 2 Stunden bei Raumtemperatur gerührt. Dann wird das ausgefallene Reaktionsprodukt abgesaugt, mit 100 ml Wasser gewaschen und getrocknet. Man erhält 17,3 g (81% d.Th.) 4-Amino-6-tert.-butyl-3-methylthio-1,2,4-triazin-5(4H)-on (VI) vom Schmelzpunkt 123–125°C.

2. Hydrolyse zur α-Ketocarbonsäure:

a) Trimethylbrenztraubensäure (IV):

17,1 g (0,1 Mol) Trimethylbrenztraubensäure-N-acetylamid (Ia) werden in 100 ml 5n-HCl 4 Stunden auf 90°C erhitzt. Nach dem Abkühlen schüttelt man mit Methylenchlorid aus, extrahiert die Methylenchloridphase mit verdünnter NaOH-Lösung, stellt die alkalische wässrige Lösung mit konzentrierter HCl auf pH1, schüttelt mit Essigester aus und dampft anschliessend den Essigesterextrakt ein. Man erhält 11,9 g (92% d.Th.) Trimethylbrenztraubensäure (IV).

b) Phenylglyoxylsäure ($C_6H_5$–CO–COOH):

Es wird wie in Beispiel B2. a) beschrieben verfahren, jedoch wird statt Trimethylbrenztraubensäure-N-acetylamid die äquivalente Menge (0,1 Mol) Phenylglyoxylsäure-N-acetylamid eingesetzt. Man erhält 13,4 g (89,3% d.Th.) Phenylglyoxylsäure.

**Patentansprüche**

1. α-Ketocarbonsäure-N-acylamide der allgemeinen Formel I

$$R^1\text{–CO–CO–NH–CO–}R^2 \qquad (I)$$

in welcher

$R^1$ für einen gegebenenfalls substituierten aliphatischen Rest mit bis zu 12 Kohlenstoffatomen, für einen gegebenenfalls substituierten Cycloalkylrest mit 3 bis 10 Kohlenstoffatomen, für einen gegebenenfalls substituierten Phenyl- oder Naphthylrest oder für einen gegebenenfalls substituierten heterocyclischen Rest steht und

$R^2$ für einen gegebenenfalls substituierten aliphatischen Rest mit bis zu 8 Kohlenstoffatomen oder für einen gegebenenfalls substituierten Phenylrest steht.

2. Trimethylbrenztraubensäure-N-acetylamid der Formel

$$(CH_3)_3C\text{–CO–CO–NH–CO–}CH_3$$

gemäss Anspruch 1.

3. Phenylglyoxylsäure-N-acetylamid der Formel

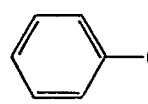CO–CO–NH–CO–$CH_3$

gemäss Anspruch 1.

4. Verfahren zur Herstellung von α-Ketocarbonsäure-N-acylamiden der allgemeinen Formel (I) gemäss Anspruch 1, dadurch gekennzeichnet, dass man Acylcyanide der allgemeinen Formel II

$$R^1\text{–CO–CN} \qquad (II),$$

in welcher

$R^1$ die oben angegebene Bedeutung hat,

mit Carbonsäureanhydriden der allgemeinen Formel III

$$R^2\text{–CO–O–CO–}R^2 \qquad (III)$$

in welcher

$R^2$ die oben angegebene Bedeutung hat,

in Gegenwart einer starken Säure und gegebenenfalls in Gegenwart eines Lösungsmittels bei Temperaturen zwischen −50 und +150°C umsetzt und das Reaktionsgemisch anschliessend mit Wasser versetzt.

5. Verfahren nach Anspruch 4, dadurch gekennzeichnet, dass man die Umsetzung bei Temperaturen zwischen 0 und 100°C durchführt.

6. Verfahren nach Anspruch 4, dadurch gekennzeichnet, dass man Acylcyanid (II) und Carbonsäureanhydrid (III) im Molverhältnis von 1:0,5-10, vorzugsweise von 1:0,8-4, besonders bevorzugt von 1:1-2 umsetzt.

7. Verfahren nach Anspruch 4, dadurch gekennzeichnet, dass man auf 1 Mol Acylcyanid (II) 0,5-10 Mol, vorzugsweise 0,8-8 Mol, besonders bevorzugt 1-4 Mol Säure einsetzt.

8. Verfahren nach Anspruch 4, dadurch gekennzeichnet, dass man Carbonsäureanhydrid (III) und Säure im Molverhältnis von 1:2 einsetzt.

9. Verfahren nach Anspruch 4, dadurch gekennzeichnet, dass man Acylcyanid (II), Carbonsäureanhydrid (III) und Säure im Molverhältnis von 1:1:2 bis 1:2:4 umsetzt.

10. Verfahren nach Anspruch 4, dadurch gekennzeichnet, dass man als Carbonsäureanhydrid (III) Essigsäureanhydrid einsetzt.

**Revendications**

1. N-acylamides d'acides $\alpha$-cétocarboxyliques de formule générale I

$$R^1\text{-CO-CO-NH-CO-}R^2 \quad (I)$$

dans laquelle
$R^1$ représente un reste aliphatique éventuellement substitué contenant jusqu'à 12 atomes de carbone, un reste cycloalkyle éventuellement substitué contenant 3 à 10 atomes de carbone, un reste phényle ou naphtyle éventuellement substitué ou un reste hétérocyclique éventuellement substitué et
$R^2$ représente un reste aliphatique éventuellement substitué contenant jusqu'à 8 atomes de carbone ou un reste phényle éventuellement substitué.

2. Le N-acétylamide de l'acide triméthylpyruvique de formule

$$(CH_3)_3C\text{-CO-CO-NH-CO-}CH_3$$

selon la revendication 1.

3. Le N-acétylamide de l'acide phénylglyoxylique de formule

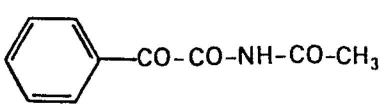

selon la revendication 1.

4. Procédé de préparation des N-acylamides d'acides $\alpha$-céto-carboxyliques de formule générale I, selon la revendication 1, caractérisé en ce que l'on fait réagir des cyanures d'acyle de formule générale II

$$R^1\text{-CO-CN} \quad (II),$$

dans laquelle
$R^1$ a les significations indiquées ci-dessus,
avec des anhydrides d'acides carboxyliques de formule générale III

$$R^2\text{-CO-O-CO-}R^2 \quad (III)$$

dans laquelle
$R^2$ a les significations indiquées ci-dessus,
en présence d'un acide fort et le cas échéant en présence d'un solvant, à des températures allant de −50 à +150°C, et on ajoute ensuite de l'eau au mélange de réaction.

5. Procédé selon la revendication 4, caractérisé en ce que l'on effectue la réaction à des températures de 0 à 100°C.

6. Procédé selon la revendication 4, caractérisé en ce que l'on fait réagir le cyanure d'acyle II et l'anhydride d'acide carboxylique III dans un rapport molaire de 1:0,5 à 10, de préférence de 1:0,8 à 4 et mieux encore de 1:1 à 2.

7. Procédé selon la revendication 4, caractérisé en ce que, pour 1 mole du cyanure d'acyle II, on utilise de 0,5 à 10 moles, de préférence de 0,8 à 8 moles, et mieux encore de 1 à 4 moles, d'acide.

8. Procédé selon la revendication 4, caractérisé en ce que l'on utilise l'anhydride d'acide carboxylique III et l'acide dans un rapport molaire de 1:2.

9. Procédé selon la revendication 4, caractérisé en ce que l'on fait réagir le cyanure d'acyle II, l'anhydride d'acide carboxylique III et l'acide dans des proportions molaires de 1:1:2 à 1:2:4.

10. Procédé selon la revendication 4, caractérisé en ce que l'anhydride d'acide carboxylique III utilisé est l'anhydride acétique.

**Claims**

1. $\alpha$-Ketocarboxylic acid N-acylamides of the general formula I

$$R^1\text{-CO-CO-NH-CO-}R^2 \quad (I)$$

in which
$R^1$ represents an optionally substituted aliphatic radical with up to 12 carbon atoms, an optionally substituted cycloalkyl radical with 3 to 10 carbon atoms, an optionally substituted phenyl or naphthyl radical or an optionally substituted heterocyclic radical and
$R^2$ represents an optionally substituted aliphatic radical with up to 8 carbon atoms or an optionally substituted phenyl radical.

2. Trimethylpyruvic acid N-acetylamide of the formula

$$(CH_3)_3C\text{-CO-CO-NH-CO-}CH_3$$

according to Claim 1.

3. Phenylglyoxylic acid N-acetylamide of the formula

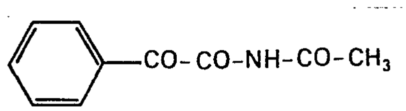

according to Claim 1.

4. Process for the preparation of $\alpha$-ketocarboxylic acid N-acylamides of the general formula (I) according to Claim 1, characterised in that acyl cyanides of the general formula II

$R^1-CO-CN$ (II)

in which
$R^1$ has the abovementioned meaning,
are reacted with carboxylic acid anhydrides of the general formula III

$R^2-CO-O-CO-R^2$ (III)

in which
$R^2$ has the abovementioned meaning,
in the presence of a strong acid and if appropriate in the presence of a solvent, at temperatures between −50 and +150°C, and water is then added to the reaction mixture.

5. Process according to Claim 4, characterised in that the reaction is carried out at temperatures between 0 and 100°C.

6. Process according to Claim 4, characterised in that the acyl cyanide (II) and carboxylic acid anhydride (III) are reacted in a molar ratio of 1:0.5–10, preferably 1:0.8–4 and particularly preferably 1:1–2.

7. Process according to Claim 4, characterised in that 0.5–10 mols, preferably 0.8–8 mols and particularly preferably 1–4 mols, of acid are employed per mol of acyl cyanide (II).

8. Process according to Claim 4, characterised in that the carboxylic acid anhydride (III) and acid are employed in a molar ratio of 1:2.

9. Process according to Claim 4, characterised in that the acyl cyanide (II), carboxylic acid anhydride (III) and acid are reacted in a molar ratio 1:1:2 to 1:2:4.

10. Process according to Claim 4, characterised in that acetic anhydride is employed as the carboxylic acid anhydride (III).